# EUROPEAN PATENT APPLICATION

(11) **EP 3 318 270 A1**
(43) Date of publication of application: **09.05.2018**
(21) Application number: 16197809.3
(22) Date of filing: 08.11.2016
(51) Int. Cl.: A61K 38/17, A61K 38/19, A61K 38/20, A61K 38/39, A61K 9/00, A61P 17/02

(54) **STATHMIN COMPOSITION AND METHOD FOR TREATING WOUNDS**

(71) Applicant: RMB-Research GmbH, 1110 Vienna (AT)
(72) Inventor: SCHUSTER, Manfred, 2191 Schrick (AT); GOMBOS, Linda, 3002 Purkersdorf (AT); WIEDERKUM, Susanne, 8240 Ortgraben (Friedberg) (AT); MAYRHOFER, Sarah, 2100 Leobendorf (AT)
(74) Representative: Sonn & Partner Patentanwälte

(57) **Abstract**

The invention provides a method of treating a wound with a composition comprising stathmin and a gel-forming component, that is in gel state at human body surface temperature; with the proviso that in case the gel-forming component comprises collagen, the gel-forming component further comprises a stiffening agent, wherein treating comprises topically depositing the composition on said wound or coating the wound, and wherein said treatment of the wound is in the presence of at least one inflammatory cytokine; as well as compositions for such a treatment.

SEQUENCE LISTING

<110>  RMB-Research GmbH

<120>  Stathmin composition and method for treating wounds

<130>  r70226

<160>  3

<170>  PatentIn version 3.5

<210>  1
<211>  149
<212>  PRT
<213>  Homo sapiens

<400>  1

Met Ala Ser Ser Asp Ile Gln Val Lys Glu Leu Glu Lys Arg Ala Ser
1               5                   10                  15

Gly Gln Ala Phe Glu Leu Ile Leu Ser Pro Arg Ser Lys Glu Ser Val
            20                  25                  30

Pro Glu Phe Pro Leu Ser Pro Pro Lys Lys Lys Asp Leu Ser Leu Glu
        35                  40                  45

Glu Ile Gln Lys Lys Leu Glu Ala Ala Glu Glu Arg Arg Lys Ser His
    50                  55                  60

Glu Ala Glu Val Leu Lys Gln Leu Ala Glu Lys Arg Glu His Glu Lys
65                  70                  75                  80

Glu Val Leu Gln Lys Ala Ile Glu Glu Asn Asn Asn Phe Ser Lys Met
                85                  90                  95

Ala Glu Glu Lys Leu Thr His Lys Met Glu Ala Asn Lys Glu Asn Arg
            100                 105                 110

Glu Ala Gln Met Ala Ala Lys Leu Glu Arg Leu Arg Glu Lys Asp Lys
        115                 120                 125

His Ile Glu Glu Val Arg Lys Asn Lys Glu Ser Lys Asp Pro Ala Asp
    130                 135                 140

Glu Thr Glu Ala Asp
145

<210>  2
<211>  174
<212>  PRT
<213>  Homo sapiens

<400>  2

Met Ala Ser Ser Asp Ile Gln Val Lys Glu Leu Glu Lys Arg Ala Ser
1               5                   10                  15

Gly Gln Ala Phe Glu Leu Ile Leu Ser Pro Arg Ser Lys Glu Ser Val
            20                  25                  30

Pro Glu Phe Pro Leu Ser Pro Pro Lys Lys Lys Asp Leu Ser Leu Glu
        35                  40                  45

Glu Ile Gln Lys Lys Leu Glu Ala Ala Glu Glu Arg Arg Lys Ser His
    50                  55                  60

Glu Ala Glu Val Leu Lys Gln Leu Ala Glu Lys Arg Glu His Glu Lys
65                  70                  75                  80

Glu Val Leu Gln Lys Ala Ile Glu Glu Asn Asn Asn Phe Ser Lys Met
                85                  90                  95

Ala Glu Glu Lys Leu Thr His Lys Met Glu Ala Asn Lys Glu Asn Arg
            100                 105                 110

Glu Ala Gln Met Ala Ala Lys Leu Glu Arg Leu Arg Glu Lys Met Tyr
        115                 120                 125

Phe Trp Thr His Gly Pro Gly Ala His Pro Ala Gln Ile Ser Ala Glu
    130                 135                 140

Gln Ser Cys Leu His Ser Val Pro Ala Leu Cys Pro Ala Leu Gly Leu
145                 150                 155                 160

Gln Ser Ala Leu Ile Thr Trp Ser Asp Leu Ser His His His
                165                 170

<210>  3
<211>  450
<212>  DNA
<213>  Homo sapiens

<400>  3
   atggcttctt ctgatatcca ggtgaaagaa ctggagaagc gtgcctcagg ccaggctttt      60
   gagctgattc tcagccctcg gtcaaaagaa tctgttccag aattccccct ttcccctcca     120
   aagaagaagg atctttccct ggaggaaatt cagaagaaat tagaagctgc agaagaaaga     180
   cgcaagtccc atgaagctga ggtcttgaag cagctggctg agaaacgaga gcacgagaaa     240
   gaagtgcttc agaaggcaat agaagagaac aacaacttca gtaaaatggc agaagagaaa     300
   ctgacccaca aaatggaagc taataaagag aaccgagagg cacaaatggc tgccaaactg 360
   gaacgtttgc gagagaagga taagcacatt gaagaagtgc ggaagaacaa agaatccaaa 420
   gaccctgctg acgagactga agctgactaa 450

## Description

The present invention relates to improved pharmaceutical compositions comprising stathmin for improving wound healing.

### Background of the invention

Stathmin is a known protein that has been described for the treatment of wounds in WO 2014/013027 A1. Also, WO 2007/089151 A1 identified stathmin as TLR3 activating substance and suggested the treatment of degenerative inflammatory processes in neurodegenerative disorders such as Alzheimer's disease. Also the treatment of wounds is mentioned.

Still there is a need for improved wound healing compositions.

### Summary of the invention

Research into stathmin effects on cells involved in wound healing has revealed that stathmin's effects in wound healing are not based on previously known intracellular effects on microtubule regulation (Mistry Sucharita J et al. (2007)) but based on a completely new effect on stimulating cellular receptors and associated pathways. Stathmin has a newly discovered mode of action on regulatory pathways in cells. It has been further found that stathmin's effects, in particular those involved in wound healing, are dependent on such regulatory pathways such as the NF-Kb pathway and these pathways are further influenced by additional factors. By supplying such additional factors, wound healing can be increased. In particular, it was found that cells involved in wound healing, such as endothelial cells, epithelial cells, fibroblasts and immune cells can be activated in the presence of inflammatory cytokines. It was further found that particular formulations can enhance stathmin's efficacy and improve cellular pathway efficiency required for wound healing. In particular phagocytosis, a necessary process during wound healing, can be improved by such formulations. The inventive formulations increase to co-action of stathmin with inflammatory cytokines to achieve efficient cell stimulation. This acting together of one or more cytokine with stathmin benefits from proper timing of these components. This is achieved by controlled release of these agents so that synergistic action of these components together can efficiently stimulate the cells.

In a first aspect, the invention provides a composition suitable for the treatment of wounds, wherein the composition comprises stathmin in a slow-release formulation of collagen and a further stiffening agent.

In a second aspect, the present invention provides a composition suitable for the treatment of wounds, wherein the composition comprises stathmin and at least one inflammatory cytokine and a gel-forming component, that is in gel state at human body surface temperature, preferably at 30-34°C. The components of the composition can also be provided in a kit-of-parts to be used in combination according to the inventive uses and methods. Also provided is the use of the composition or kit in wound healing or the use in the manufacture of a medicament for wound healing.

In a further aspect, the invention provides a method of treating a wound with a composition comprising stathmin and a gel-forming component, that is in gel state at human body surface temperature or at 30-34°C, wherein treating comprises topically depositing the composition on said wound or coating the wound, and wherein said treatment of the wound is in the presence of at least one inflammatory cytokine.

The following detailed disclosure reads on all aspects and embodiments of the present invention, irrespective of relating to a method, composition, kit or use. E.g. described method steps also disclose that composition or kit has elements for its suitability in the method. Elements described for the composition or kit can read on uses of such a composition or kit for the inventive methods.

### Detailed description of the invention

The present invention provides a composition suitable for the treatment of wounds, wherein the composition comprises stathmin in a slow-release formulation of collagen and a further stiffening agent. Also provided is a similar composition comprising stathmin and at least one inflammatory cytokine and a gel-forming component, that is in gel state at human body surface temperature or at 30-34°C, e.g. at 34°C or event at 37°C; with the proviso that in case the gel-forming component comprises collagen, the gel-forming component further comprises a stiffening agent. Both definitions of composition improve the suitability of stathmin effectively treating a wound. The first composition has a preferred gel-forming component in the form of collagen and a further stiffening agent, which is of course also a preferred embodiment of the second composition. The second composition further defines the presence of an inflammatory cytokine. As shown by the present invention, an inflammatory cytokine is necessary for the improved action of stathmin. However, the cytokine is not necessarily supplied to a wound since in many wounds inflammatory cytokines are already present. Thus a composition without the cytokine is fully functional according to the invention. This is achieved by the slow-release (also referred to as extended release) of stathmin that allows triggering of cellular pathways that depend on the presence of the inflammatory cytokine and stathmin. Of course, the inflammatory cytokine can also be included in the first composition. Consequently the delimitations of the first and second composition are fluid and solely related to these minor differences in formulations. All preferred aspects of the composition described herein read on both compositions likewise.

Also provided is a kit comprising stathmin and at least one inflammatory cytokine and a gel-forming component, that is in gel state at human body temperature or at 30-34°C, e.g. 34°C; with the proviso that in case the gel-forming component comprises collagen, the gel-forming component further comprises a stiffening agent; wherein stathmin is dispersed in the gel-forming component.

Stathmin is a protein with known sequence (NCBI database NP_005554 and CAD19057 or SEQ ID NO: 1 (stathmin a) or SEQ ID NO: 2 (stathmin b)). An encoding nucleic acid sequence is provided as SEQ ID NO: 3. Stathmin and its isoforms are further known from the background art mentioned above. It is a ubiquitously expressed protein found in most tissues in mammals. Many different phosphorylated forms are observed depending on specific combinations among the sites, which can be phosphorylated. Phosphorylation at Ser-16 seems to be required for neuron polarization. Phosphorylation at Ser-63 reduces tubulin binding 10-fold and suppresses the MT polymerization inhibition activity. Stathmin, as used herein relates to any stathmin isoform or variant or any post-translationally modified form, including phosphorylated forms. Stathmin as used according to the invention may or may not be phosphorylated. In preferred embodiments stathmin comprises amino acids 1 to 126 of SEQ ID NO: 1, or an amino acid sequence that has at least 70 % sequence identity with the sequence of amino acids 1 to 126 of SEQ ID NO: 1. The amino acids 1 to 126 of SEQ ID NO: 1 are also found in SEQ ID NO: 2 and appear to be of a homologous region. Further preferred stathmin variants are disclosed in WO 2007/089151, US 6,429,304 B1, WO 2004/113561 A2 and NCBI database entries NP_005554 and CAD19057 (all incorporated herein by reference).

The term "sequence identity" refers to identity between two sequences, usually amino acid sequences, which can be determined by sequence alignment programs like BLAST, PSI-BLAST (www.ncbi.nlm.nih.gov/blast/) or ClustalO (http://www.ebi.ac.uk/Tools/msa/clustalo/). These algorithms calculate the best match for the selected sequences, and line them up so that the identities, similarities and differences can be seen.

In preferred embodiments of the present invention the inventive stathmin comprises a sequence with at least 75 %, more preferred at least 80 %, at least 85%, at least 90%, at least 95%, at least 98% or even 100%, sequence identity with the sequence of amino acids 1 to 126 of SEQ ID NO: 1. In even more preferred embodiments the inventive stathmin comprises a sequence with at least 70 %, more preferred at least 75%, at least 80 %, at least 85%, at least 90%, at least 95%, at least 98% or even 100%, sequence identity with the sequence as set forth in SEQ ID NO: 1. Stathmin may be stathmin-1, stathmin-2, stathmin-3 or stathmin-4, preferably it is stathmin-1. Stathmin can be a recombinant stathmin. Further preferred it is of the same origin as the patient but also stathmin variants from other animals can be used. Preferably the patient is a mammal, especially a human or non-human animal, in particular a domestic animal, such as pig, rodents, or a primate. Preferably the stathmin is human stathmin or stathmin from a non-human animal, in particular a domestic animal, such as pig, rodents, or a primate.

As used herein "comprising" is used in an open meaning, i.e. that the stathmin of the present invention may have further amino acids or protein components. It may be a fusion protein. Such extended stathmin proteins may have in preferred embodiments a limited size, e.g. up to 2000 amino acids, up to 1800 amino acids, up to 1600 amino acids, up to 1400 amino acids, up to 1200 amino acids, up to 1000 amino acids, up to 800 amino acids, up to 600 amino acids, up to 400 amino acids, up to 300 amino acids, up to 200 amino acids, or up to 160 amino acids. Of course the invention also relates to stathmin proteins that consist of any one of said sequences comprised in the above mentioned embodiments. "Consisting" is used in a closed and sequence limiting meaning.

In preferred embodiments stathmin is phosphorylated. Phosphorylation is a natural post-transcriptional process and occurs during recombinant expression. Possible phosphorylation sites are at amino acids corresponding to amino acids 16, 25, 28, 38, 63, 146 of SEQ ID NO: 1. Preferred phosphorylation are at amino acids corresponding to amino acids 16 and/or 63 of SEQ ID NO: 1. The inventive stathmin may comprise 1, 2, 3, 4, 5 or 6 amino acid phosphorylations.

In preferred embodiments stathmin is acetylated. Acetylation is a natural post-transcriptional process and occurs during recombinant expression. Possible acetylation sites are at amino acids corresponding to amino acids 2, 9, 80, 95, 100, 119, 128 of SEQ ID NO: 1. The inventive stathmin may comprise 1, 2, 3, 4, 5, 6 or 7 amino acid acetylations.

The stathmin can be glycosylated or not glycosylated. E. coli produced stathmin is not glycosylated and still effective.

The inventive composition comprises a gel-forming component or agent. The gel-forming component can be any biocompatible matrix, such as chitosan, alginate, xanthan, hydroxyethylcellulose chitosan and/or hyaluronan, polyacrylate, polymethacrylate but preferred is collagen for all embodiments of the invention. It can be a hydrofiber, a hydrogel, a hydrocolloide or a foam.

Alginate gels are available commercially, e.g. as AlgiSite, Comfeel, Curasorb, Kaltogel, Kaltostat, Sorbsan, Tegagel. Alginate is made from seaweed extract and contains guluronic and mannuronic acids that provide tensile strength. Calcium and sodium alginates confer an absorptive capacity. Some can leave fibers in the wound if they are not thoroughly irrigated.

Hydrofibers are e.g. available as Aquacel, Aquacel-Ag, Versiva. Hydrofibers are an absorptive textile fiber pad, hydrofiber is also available as a ribbon for packing of deep wounds. This material is covered with a secondary dressing. The hydrofiber combines with wound exudate to produce a hydrophilic gel.

Hydrogels are e.g. Aquasorb, DuoDerm, Intrasite Gel, Granugel, Normlgel, Nu-Gel, Purilon Gel, KY Jelly. Hydrogel dressings are water-based or glycerin-based semipermeable hydrophilic polymers. They may have cooling properties to decrease wound pain. These gels can lose or absorb water depending upon the state of hydration of the wound. Hydrogels comprise a network of polymer chains that are hydrophilic, sometimes found as a colloidal gel in which water is the dispersion medium. Hydrogels are highly absorbent (they may contain 99.9% water) natural or synthetic polymers. Hydrogels also possess a degree of flexibility very similar to natural tissue, due to their significant water content. They provide a reservoir in topical drug delivery.

Hydrocolloids are e.g. CombiDERM, Comfeel, DuoDerm CGF Extra Thin, Granuflex, Tegasorb. Hydrocolloid dressings are made of microgranular suspension of natural or synthetic polymers, such as gelatin or pectin, in an adhesive matrix. The granules change from a semihydrated state to a gel as the wound exudate is absorbed. Foams like LYOfoam, Spyrosorb, Allevyn are also possible.

Collagen is the most preferred gel-forming component. It can comprise or consist of type I collagen, type II collagen, type III collagen or combinations thereof. Preferred is type I collagen since it is best associated with skin and scar tissue and hence is best associated with wound healing. Also preferred is a mixture of types I and III collagen, which together are a good supplement for skin.

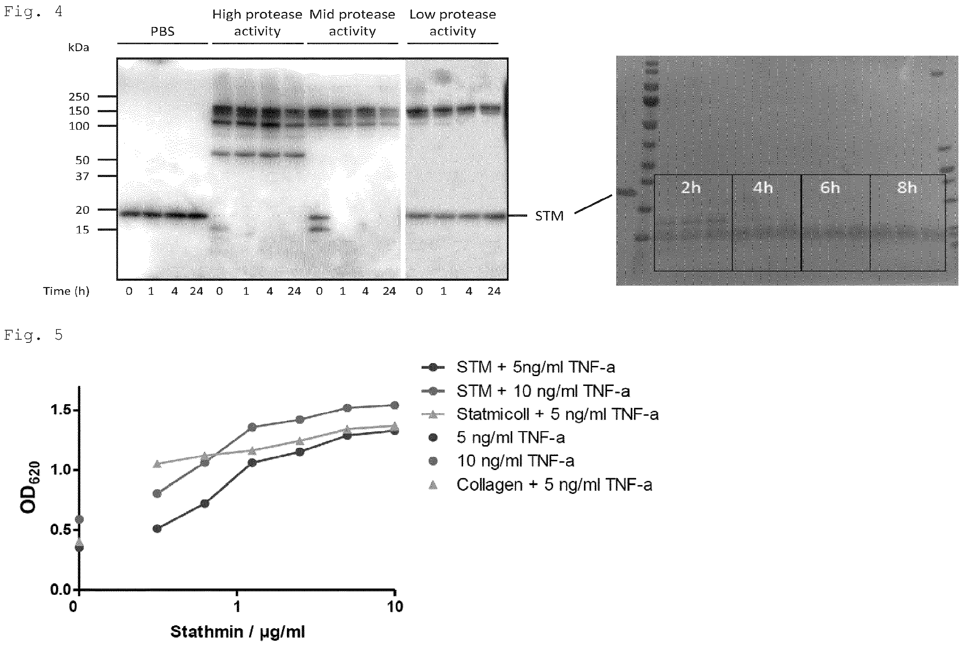

Another reason of collagen being the most preferred gel-forming component is its ability to occupy proteases, such as collagenases. As shown herein, stathmin can be degraded by proteinases, including collagenases (Fig. 4). By including collagen, stathmin can be protected from degradation. In case of other gel-forming components, it is preferred to include one or more proteinase or collagenase inhibitor or take other measures to protect stathmin from degradation by proteinases or collagenases, such as those found in a wound.

Collagen is not entirely suitable for slow-release or extended release compositions since at body temperature, including body surface temperatures, it may liquefy or release stathmin at a higher rate than required. As will be discussed in more detail below, it is intended to release stathmin over an extended period of several hours, e.g. at least 16h, to facilitate proper triggering of regenerative or inflammatory pathways necessary in a wound. Therefore collagen is preferably used together with a stiffening agent that decreases the gel forming temperature of collagen. Such a stiffening agent can be a gel-forming component by itself and may be selected from the choices listed above (e.g. chitosan, alginate, xanthan, hydroxyethylcellulose chitosan and/or hyaluronan, polyacrylate, polymethacrylate, hydrofiber, a hydrogel, a hydrocolloide or a foam). Its function is to preserve gel-properties of the composition and to protect the slow-release/extended release properties of the composition in total when applied to a wound. The composition shall be in gel state at a temperature of at least 30°C, 31°C, 32°C, 33°C, 34°C, 35°C, 36°C, 37°C, 38°C, 39°C, 40°C, 41°C, 42°C, 43°C, 44°C, 45°C or more or at any interval in between these values. Especially preferred, the stiffening agent is polymethacrylate, which fulfils these requirements, is a good mediator of slow/extended release and has very good biocompatibility. Another possibility to the addition of another agent, like a further gel-forming component is to increase the gel-forming temperature of collagen itself. Thereby collagen can be made harder or increase its stiffness. This can be done by using cross-linked collagen. Accordingly, the stiffening agent is a collagen-crosslinker. Such a crosslinker is preferably a covalently bound cross-linker. Such a cross-linker is e.g. riboflavin or a phenylenediamine that may activate collagen to form a cross-link with or without integration into the cross-linking bond.

The gel-forming component, in particular the stiffening agent, mediates slow-release of stathmin and if a cytokine is present in the composition, preferably also of the cytokine. Slow-release, also referred to as extended release, preferably refers to a continuous release for at least 8 hours (h), preferably at least 10h, at least 12h, at least 14h, at least 16h, at least 18h, at least 20h, at least 22h or at least 24h, when applied topically to a wound. The extended release shall be at a concentration of stathmin to trigger the NF-kB pathway. Preferably the concentration of released stathmin during this period is at least 0.1 µg/ml, at least 0.5 µg/ml, preferably at least 1 µg/ml, 2 µg/ml, 4 µg/ml, 6 µg/ml, 8 µg/ml or 10 µg/ml or more or any range in between these values. The stathmin release may be at a concentration of up to 500 µg/ml or even more, preferably it is up to 400 µg/ml, up to 300 µg/ml, up to 200 µg/ml, up to 100 µg/ml, up to 90 µg/ml, up to 50 µg/ml, up to 40 µg/ml, up to 30 µg/ml, or any range in between these values. Release may be in a concentration of 1 µg/ml to 500 µg/ml for at least 8 hours, for example or 0.5 µg/ml to 100 µg/ml for at least 16h.

In order to control this release, the concentration of the gel-forming component, e.g. collagen and the stiffening agent, is adequately selected. Such releases can be tested by simple release tests. In preferred embodiments, the concentration of all gel-forming components together, and including collagen, in the composition is 0.25% to 30%, preferably the concentration of all gel-forming components is 0.35% to 20%, more preferred 0.5% to 15%, 0.75% to 12% 1% to 10%, preferably about 1.5% to 8% or about 2% (all %-values in w/v).

Preferably the concentration of collagen in the composition is 0.25% to 15%, preferably the concentration of collagen is 0.35% to 12%, more preferred 0.50% to 10%, 0.75% to 8%, or 0.9% to 5%, preferably about 1% (all %-values in w/v).

Preferably the concentration of the stiffening agent (a gel-forming component used in addition to collagen) in the composition is 0.25% to 15%, preferably the concentration of the stiffening agent is 0.35% to 12%, more preferred 0.50% to 10%, 0.75% to 8%, or 0.9% to 5%, preferably about 1% (all %-values in w/v).

A preferred composition has equal concentration collagen and stiffening agent, e.g. about 2% (w/v), more preferably about 1% (w/v), each. "About" refers to +/-20%.

The composition usually maintains stathmin in an aqueous medium, e.g. at physiological osmolarity (e.g. ionic strength of 0.9% NaCl, or a osmolarity of 200-400 milli-osmoles per kilogram, preferably 250-350 milli-osmoles per kilogram, or 275-305 milli-osmoles per kilogram). The pH may be between 4 and 9, preferably between 5 and 8 or between 6 and 7.

The composition may have higher stathmin concentrations to achieve extended release of these releases concentration. This may be necessary if the composition is further diluted by wound or any other body fluid. Accordingly, the composition may comprise stathmin in solution of 10 µg/ml to 800 µg/ml, preferably 15 µg/ml to 600 µg/ml, or 20 µg/ml to 400 µg/ml.

As stated above a cytokine is necessary to improve the action of stathmin in a wound. Usually cytokines are present at sufficient concentrations in a wound. It is of course possible to add a cytokine or a compound that stimulates cytokine release from cells found in wounded tissue to be treated to the composition. Possible cytokines are TNF-alpha, IL-1-alpha, IL-1-beta and IL-8. A most preferred cytokine is TNF-alpha. The cytokine may be added to the composition in a concentration of 0.5 ng/ml to 50 ng/ml, preferably of 1 ng/ml to 25 ng/ml or of 5 ng/ml to 10 ng/ml.

The composition may also comprise a collagenase inhibitor or substrate to protect stathmin from proteolytic degradation, preferably wherein the collagenase substrate is collagen.

Furthermore, the composition may comprise a disinfectant, an anti-septic compound, a microcidal compound or a bactericidal compound. An example compound is silver. Ionic silver that has strong antimicrobial properties against many organisms, including methicillin-resistant *Staphylococcus aureus* and vancomycin-resistant enterococci. Other compounds are anti-septics like chlorhexidine or octenidine. The disinfectant, an anti-septic compound, a microcidal compound or a bactericidal compound preferably does not interfere with wound healing and maintains stability of stathmin and collagen in the composition and in the wound.

The composition may further comprise an acceptable diluent, carrier, solubilizer, emulsifier, preservative and/or adjuvant. Preservatives are e.g. thimerosal or benzyl alcohol or an anti-septic mentioned above.

The composition can be used in the treatment of wounds, wherein the composition is applied topically on a wound, wherein the composition is in gel state and optionally remains in gel state on the wound for at least 1 hour, preferably for at least 8h, at least at least 10h, at least 12h, at least 14h, at least 16h, at least 18h, at least 20h, at least 22h or at least 24h, when applied topically to a wound.

The composition preferably is included in a patch for delivery and may have a fabric support. The composition is preferably provided in a reservoir layer of the path having a backing layer which protects the depot layer from the environment. On the release side, opposite of the backing layer, a fabric support may be present that allows transfer of stathmin from the composition through this fabric layer for application. The fabric layer may also have slow-release or delayed release properties. In other embodiments, no fabric layer is used and the depot layer is placed directly on a wound. For storage a protection layer may be placed on the fabric layer or on the depot layer, that may be removed prior to use.

In other embodiments, the composition may be directly applied to a wound, e.g. brushed on or laid on the wound or coating the wound as a cream.

The invention also provides a method of treating a wound with a composition comprising stathmin and a gel-forming component, that is in gel state at human body surface temperature, preferably at 30-34°C, e.g. 34°C. The composition is defined as above. In case the gel-forming component comprises collagen, the gel-forming component further comprises a stiffening agent. Treating may comprise topically depositing the composition on said wound or coating the wound. Said treatment of the wound is in the presence of at least one inflammatory cytokine - as mentioned above, preferably TNF-alpha, or IL-1a or IL-1b. The cytokine may be present in the wound or it may be supplied to the wound artificially, e.g. but not necessarily in the inventive composition. It may also be administered separately. Therefore the inventive kit also may comprise the cytokine separately form the composition.

The wound to be treated is preferably a skin wound.

The treating method may also comprise a test to confirm if a wound to be treated has sufficient cytokines present. Therefore the presence of one or more inflammatory cytokines, preferably selected from TNF-alpha, IL-1-alpha, IL-1-beta and IL-8, are tested in wound fluid. Usually a concentration of 0.1 ng/ml of any one of these cytokines is a sufficient indicator that the stathmin composition will sufficiently promote wound healing.

The patient to be treated is preferably a human or a non-human animal. Stathmin and/or the cytokine are preferably from the same species as the species to be treated.

The wound may be a chronic or an acute wound. A chronic wound shows no or limited wound healing. It is a wound that does not heal in an orderly set of stages and in a predictable amount of time. In particular embodiments, the chronic wound is a wound that has no or reduced wound healing (e.g. only about 20 % wound area closure) within 90 days. In preferred embodiments the wound is anoxic and/or lacks sufficient oxygen supply and/or lacks newly formed arteries. The present invention also stimulates angiogenesis and can help to restore proper or even accelerated wound healing as well as angiogenesis, in a wound or elsewhere.

An acute wound is a wound that is not older than 90 days or if older that shows wound healing and thereby is not a chronic wound.

Alternatively or in combination, the wound is a wound with disease-dependent impaired wound healing (disease-dependent impaired wound healing can also be chronic as defined above). Disease-dependent impaired wound healing can be due to a disease selected from diabetes or metabolic syndrome, chronic venous insufficiency (CVI), peripheral artery occlusive disease (PAOD), cancer, autoimmunity, especially an autoimmunity selected from rheumatoid arthritis, lupus (in particular systemic lupus erytematodes) and livedoid vasculopathy, surgical wounds, ostomy, prolonged inflammatory processes, cellular senescence such as in decubitus growth factor deficiency or growth factor receptor deficiency. The patient treated according to the invention may have one or more of these diseases or conditions. Preferably said patient suffers from diabetes or metabolic syndrome.

Alternatively or in combination, the wound is a wound with medication-dependent impaired wound healing. Medication-dependent impaired wound healing can be due to a medication selected from treatments with a corticosteroid, nicotine, an antibiotic, an immunosuppressant, an anti-coagulant, a cytotoxic medication, an anti-rheumatism-medication, especially an anti-rheumatoid arthritis medication, a vasoconstrictor. The patient treated according to the invention may have received one or more of these medications.

Thus the present invention also provides for the use of stathmin for stimulating angiogenesis. A patient with tissues with insufficient artery supply may be treated. The patient may have anoxic tissues that are treated by the present invention. The tissue with insufficient artery supply may also be chronic, e.g. be in this state for 14 days, preferably 18 days, especially preferred 22 days, even more preferred 28 days, for 34 days, 40 days, 50 days or even 60 days, preferably 90 days.

Chronic diseases may have acute causes, such as surgical or accidental wounds, or chronic causes, such as in diabetes, venous insufficiency, decubitus or other comorbidities, especially a combination with other diseases that cause reduced oxygenation of the skin or other tissues.

Thus, the present invention relates to a treatment of a patient who is in need of an induction of angiogenesis with stathmin, in particular in the tissue in need of such therapy. Preferably the therapy is topical (as is preferred for all embodiments of the invention).

In preferred embodiments the patient does not suffer from a systemic degenerative inflammatory process. The treated wound may or may not comprise a degenerative inflammatory process.

Further treatments are of patients who are in need of a stimulation of the innate immune response and/or in need of an induction of cytokines/growth factors and/or TIMPs for immune modulation. Such patients may have a locally or systemic immune deficiency. The immune deficiency may be an insufficient supply (locally or systemically) of all immune cells or a limited immune cell activation or a limited supply (locally or systemically) of particular immune cells, especially cells of the innate immune system, such as NK cells, which are preferably stimulated according to the invention. Locally preferably relates to the wounded area. Preferably the immune deficiency is a deficiency in cell-mediated immunity and/or deficiency in the innate immune system. The immune system is divided into a more primitive innate immune system, and acquired or adaptive immune system, each of which contains humoral and cellular components.

The composition can be used to treat a wound in a patient who suffers from diabetes or metabolic syndrome. Diabetes and the metabolic syndrome cause a dysregulation in the patients proliferative and metabolic capabilities, which leads to reduced or no wound healing. Further, diabetes causes immune compromise and damage to small blood vessels, preventing adequate oxygenation of tissue, which can cause chronic wounds even without acute causes.

Particular examples of wounds or conditions that can be treated with stathmin are wounds selected from diabetic foot wound, especially a diabetic foot ulcer, an ulcer in general, in particular venous ulcer, a decubitus or pressure ulcer, burns, preferably a third degree burn, a surgical wound, an accidental wound, a necrotic wound, an infected wound. These wounds and conditions may be chronic or acute. In preferred embodiments, chronic or acute versions of all of these wounds and conditions are treated according to the invention.

The present invention will be further explained by the following figures and examples without being limited to these specific aspects of the invention.

### Figures:

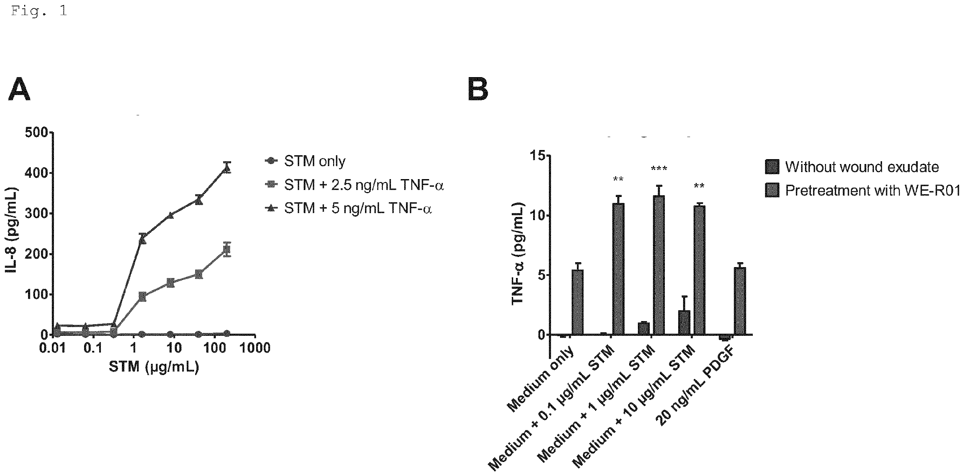

**Figure 1****.** Improved effects of stathmin on cytokine production. **A.** IL-8 secretion by the A431 epithelial cell line. Stathmin (STM) was applied at concentrations up to 200 µg/mL alone or in combination with two different concentrations of TNF-α for 16 h. IL-8 concentrations were measured by a commercially available ELISA kit (eBioscience). Shown are representative results; N=3, mean±SD. **B.** TNF-α secretion by primary human macrophages. Stathmin (STM) was applied at three different concentrations (0.1-10 µg/mL); platelet-derived growth factor (PDGF) was used as positive control. The treatment was carried out in the absence or presence of a chronic wound exudate (WE-R01) for 24 h. N=3, mean±SEM. One-way ANOVA was carried out comparing between different treatment groups. Significant differences compared to the samples which have not received stathmin treatment are indicated; **, p≤0.01; ***, p≤0.001.

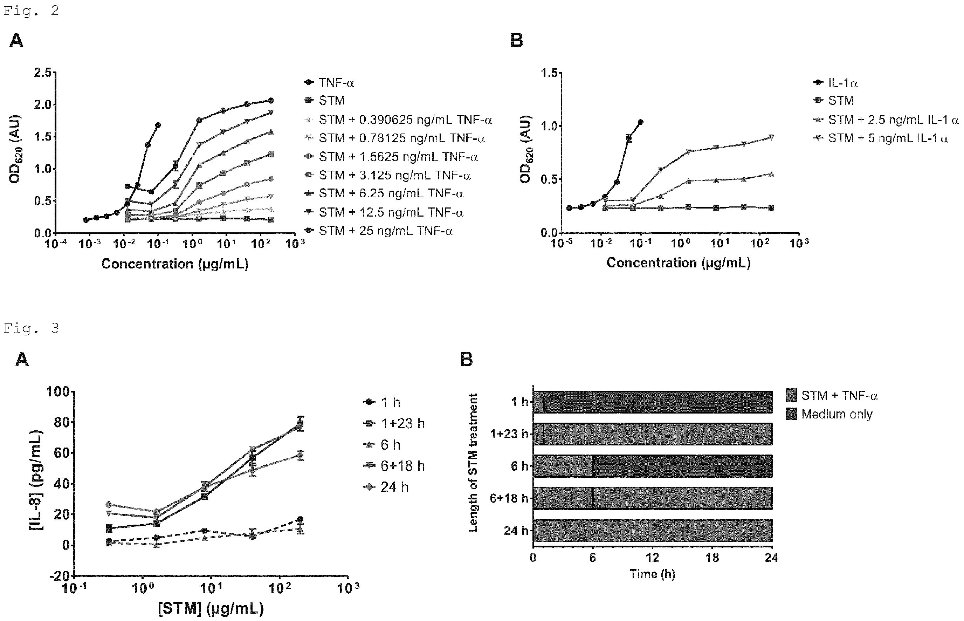

**Figure 2****.** HEK-Blue Null 1 cells (InvivoGen) were cultivated in DMEM medium in presence of 10% (v/v) FCS, 50 U/ml Penicillin, 50 mg/ml streptomycin and 100 µg/ml Zeocin (InvivoGen) at 37°C and 5% CO₂. Cells were split at a maximal confluency of 70%. Serial dilutions of Stathmin alone or in combination with collagen (Stamicoll) were prepared at a ten times higher concentration in presence **A** TNF-α or **B** IL-1α (Tonbo). 20 µl of these samples were transferred onto the 96-well assay plate. HEK-Blue Null1 cells were washed with - and resuspended - in 37°C warm PBS. Cells were counted and diluted to a density of 280,000 cells/ml with HEK-Blue detection medium (InvivoGen). 180 µl of this cell suspension (50,000 cells) were transferred to each well of the cell culture plate, which was incubated over night at 37°C and 5% CO₂. Color of the supernatant was measured with a microplate reader (Tecan Sunrise) at 620 nm.
**Figure 3****.** A431 cells were cultivated in DMEM medium supplemented with 10% (v/v) FCS, 50 U/ml penicillin and 50 mg/ml streptomycin at 37°C and 5% CO₂. 20.000 cells were seeded per well of a 96-Well cell culture plate. The plates were incubated overnight in presence of 2.5 ng/ml TNF-α. They were washed with PBS and incubated with serial dilutions of Stathmin in presence of 2.5 ng/ml TNF-α, in absence of PBS. Wells were washed with PBS after 1 h, 6 h and 24 h and incubated again in medium of the same composition (control) or in DMEM without FCS until 24 h (Timing scheme shown in **B**). 100 µl of the supernatant were investigated for IL8 levels using the human IL-8 ELISA Ready-SET-Go!® (2nd Generation) (eBioscience) according to the instructions of the manufacturer. Results shown left **(A).**
**Figure 4****. left.** Proteolytic stability of Stathmin was analyzed at a concentration of 0.1 mg/ml in PBS in presence or absence of 10% (v/v) wound-fluid of chronic wounds from different donors. These wound fluids showed high, medium or low proteolytic activity. Samples were taken immediately after incubation on ice, and after incubation for 1, 4 and 24 hours at 37°C. They were mixed immediately with SIGMAfast Protease Inhibitor Cocktail and stored at -80°C until being analyzed. Then, they were diluted 1:10 in XT Sample Buffer (Bio-Rad) and heated for 10 min at 90°C. 10 µl were loaded on a Criterion XT 4-12% Bis-Tris Gel (Bio-Rad) which was run according to the instructions of the manufacturer. Proteins were then blotted on a PVDF membrane in a Semidry-Blot-System at 0.24 A per gel for 1 h. The membrane was blocked in 5% skimmilk powder in PBST (0.05% Tween 20 in PBS) and incubated with an Stathmin specific antibody Op18(FL-149) (Santa Cruz) at a 1:200 dilution in 5% skimmilk powder. Detection was performed with the HRP conjugated goat anti rabbit antibody (Santa Cruz) in a 1:5000 dilution in combination with the Clarity Western ECL substrate (Bio-Rad). **right.** Proteolysis of Stathmin was studied at 0.1 mg/ml in presence of 50 U/ml collagenase in 100 mM Na-phosphate (pH 7.0) for 8 h at 37°C and subsequent Coomassie stained SDS-PAGE.
**Figure 5****.** Activation of HEK cells in stathmin with 5 ng/ml (lower circles) or 10 ng/ml (upper circles) TNF-alpha or with a formulation of stathmin in 2% collagen, 2% polymethacrylate (Carbopol Ultrez 10NF) and 20 µg/ml stathmin (STM in collagen). Stathmin in collagen and polymethacrylate surpasses the effects of stathmin alone.

### Examples:

A431 cells were grown in DMEM growth medium supplemented with 10% FCS o/n (37°C; 5% CO₂) in a 96 well plates at cell densities of 2*10E4 cells/well. The next day the cells were treated with different concentrations of STM (200µg/ml; 40µg/ml; 8µg/ml; 0.32µg/ml and 0.064µg/ml) in serum free DMEM supplemented with 2.5ng/ml TNF a. Cells were incubated for 1, 6 or 24 hours. In the first cases, they were washed with PBS and incubated in medium without FCS for the remaining incubation time. Cells which were incubated continuously for 24 hours with medium served as a control.

Stathmin alone evokes only a limited cellular response in any of the studied cell types (epithelial cells, fibroblasts, endothelial cells, immune cells etc.). Additional stimulation through another receptor transducing a stress signal is required for improved effects (Figure 1A). This means that stathmin is highly specific for stimulated cells under stress and does not have an effect on cells under normal conditions. Thus stathmin as active ingredient will have very little side effects. We confirmed the improved effects of stathmin using different co-stimuli including chronic wound exudate (Figure 1B).

A preparation of stathmin with collagen and polymethacrylate was prepared. A 2% collagen suspension is dissolved at room temperature at a pH of 4.0 in 5 mM HCl and stored at 2-8°C overnight. This is then brought to room temperature and adjusted to a pH of 6.5 with 1 M NaOH. Stathmin is added to 20 µg/ml in the final dosage form. Polymethacrylate (Carbopol Ultrez 10NF) is autoclaved as a 2% aqueous solution at 125°C for 20 minutes and buffered with 10 mM Na₂HPO₄ at a pH of 6.0. The dosage form of stathmin consists in equal parts of a collagen and polymethacrylate in suspension as described. It is stored at 2-8°C until application. As shown in figure 5, the dosage form shows a markedly increased biological effect in cellular experiments. In the presence of 5 ng/ml TNF-α, the concentration-dependent activation of HEK cells of the dosage form has higher activity by a factor of more than 2 as compared to a stathmin solution of the same active substance concentration. The activation by the same TNF-α concentration without stathmin is obviously much lower (shown directly on y-Axis of Fig. 5). This amazing synergy effect is seen in addition to increased proteolytic stability (Fig. 4) and extended release/treatment (Fig. 3).

## Claims

1. A composition suitable for the treatment of wounds, wherein the composition comprises stathmin in a slow-release formulation of collagen and a further stiffening agent.

2. A composition suitable for the treatment of wounds, wherein the composition comprises stathmin and at least one inflammatory cytokine and a gel-forming component, that is in gel state at 34°C; with the proviso that in case the gel-forming component comprises collagen, the gel-forming component further comprises a stiffening agent.

3. The composition according to claim 2, wherein the inflammatory cytokine is selected from TNF-alpha, IL-1-alpha, IL-1-beta and IL-8.

4. The composition according to claim 2 or 3, wherein the gel-forming component is a composite of collagen and a stiffening agent that increases the gel forming temperature of collagen.

5. The composition according to claim 1 or 3, wherein the stiffening agent is a gel-forming polymer or a collagen-crosslinker, alginate, a hydrofiber, a hydrogel, a hydrocolloide or a foam, preferably wherein the gel-forming polymer is selected from polymethacrylate.

6. The composition of any one of claims 1 to 5, wherein the gel-forming component mediates slow-release of stathmin and the cytokine, preferably wherein slow-release is a continuous release for at least 16 hours when applied topically to a wound.

7. The composition of any one of claims 1 to 6, wherein the composition releases stathmin in a concentration of 1 µg/ml to 500 µg/ml for at least 8 hours.

8. The composition of any one of claims 1 to 7, wherein the composition comprises stathmin in solution of 10 µg/ml to 800 µg/ml.

9. The composition of any one of claims 1 to 8, wherein the cytokine is in a concentration of 0.5 ng/ml to 10 ng/ml.

10. The composition of any one of claims 1 to 9, comprising a collagenase inhibitor or substrate to protect stathmin from proteolytic degradation, preferably wherein the collagenase substrate is collagen.

11. Kit comprising a stathmin and at least one inflammatory cytokine and a gel-forming component, that is in gel state at human body temperature, preferably at 34-37°C; with the proviso that in case the gel-forming component comprises collagen, the gel-forming component further comprises a stiffening agent; wherein stathmin is dispersed in the gel-forming component.

12. The composition or kit according to any one of claims 1 to 11 for use in the treatment of wounds, wherein the stathmin is applied topically on a wound, wherein the stathmin is in gel and optionally remains in gel state on the wound for at least 1 hour.

13. The method of treating a wound with a composition comprising stathmin and a gel-forming component, that is in gel state at human body temperature, preferably at 30-34°C; with the proviso that in case the gel-forming component comprises collagen, the gel-forming component further comprises a stiffening agent; wherein treating comprises topically depositing the composition on said wound or coating the wound, and wherein said treatment of the wound is in the presence of at least one inflammatory cytokine; preferably wherein the composition is defined in any one of claims 1 to 10.

14. A composition as defined in claim 13 for use in the method of claim 13.

15. The composition for use or method according to any one of claims 11 to 14, wherein the wound is a chronic or an acute wound.
